# EUROPEAN PATENT APPLICATION

(11) **EP 1 930 432 A1**
(43) Date of publication of application: **11.06.2008**
(21) Application number: 06797579.7
(22) Date of filing: 31.08.2006
(51) Int. Cl.: C12N 15/09, C12C 11/02, C12G 1/00, C12N 1/19, C12Q 1/02, C12Q 1/68

(54) **TRYPTOPHAN TRANSPORTER GENE AND USE THEREOF**

(30) Priority: 01.09.2005 JP 2005253271; 27.02.2006 JP 2006051367
(71) Applicant: SUNTORY LIMITED, Osaka-shi, Osaka 530-8203 (JP)
(72) Inventor: NAKAO, Yoshihiro, c/o SUNTORY LIMITED, Research Center, Mishima-gun, Osaka 6188503 (JP); KODAMA, Yukiko, c/o SUNTORY LIMITED, Resaerch Center, Mishima-gun, Osaka 6188503 (JP); SHIMONAGA, Tomoko, c/o SUNTORY LIMITED, Research Center, Mishima-gun, Osaka 6188503 (JP)
(74) Representative: Vossius & Partner
(86) International application number: PCT/JP2006/317703
(87) International publication number: WO 2007/026958

(57) **Abstract**

The present invention relates to a tryptophan transporter gene and to uses of the gene. The invention relates in particular to a brewer's yeast which can control the tryptophan assimilation ability, alcoholic beverages produced using such yeast, and a method of producing such alcoholic beverages. More specifically, the invention relates to a yeast which can control the tryptophan assimilation ability by controlling the level of expression of the TAT2 gene which codes for the tryptophan transporter Tat2p in brewer's yeast, particularly of the nonScTAT2 gene characteristic to beer yeast, and to a method of producing alcoholic beverages using such yeast.

## Description

### TECHNICAL FIELD

The present invention relates to a tryptophan transporter gene and to uses of the gene. The invention relates in particular to a brewer's yeast which can control the tryptophan assimilation ability, alcoholic beverages produced using such yeast, and a method of producing such alcoholic beverages. More specifically, the invention relates to a yeast which can control the tryptophan assimilation ability by controlling the level of expression of the TAT2 gene which codes for the tryptophan transporter Tat2p in brewer's yeast, particularly of the nonScTAT2 gene characteristic to beer yeast, and to a method of producing alcoholic beverages using such yeast.

### BACKGROUND ART

In general, amino acids are important as taste components of alcoholic beverages and are known to be critical elements governing the quality of the products. Thus, it is important for developing a novel type of alcoholic beverage to control the amino acid content according to the quality of the alcoholic beverage of interest.

However, amino acids are assimilated by yeast as nitrogen sources during fermentation, and it is extremely difficult to control the amino acid content at the completion of fermentation.

To utilize extracellular amino acids as nitrogen sources, the amino acids must be transported into the yeast cells. It has been demonstrated that amino acid transporters present in the yeast cell membrane are responsible for the transport of the amino acids.

As yeast amino acid transporters, Gapl, with low substrate specificity, and a large numbers of other amino acid transporters having different substrate specificity are known, including the tryptophan transporter TAT2, the arginine transporter Can1 and the proline transporter Put4 (Mol Cell Biol. 14: 6597-6606, 1994; Curr Genet 36: 317-328, 1999).

Examples have hitherto been reported in which a yeast mutant having mutations in the genes involved in the transport of amino acids (gap1, shr3, can1, put4 and uga4) was used for controlling the amino acid content in alcoholic beverages (Japanese Examined Patent Publication (Kokai) No. 2001-321159), and in which the branched-chain amino acid transporter BAP2 was highly expressed (Japanese Examined Patent Publication (Kokai) No. 2000-316559).

### DISCLOSURE OF INVENTION

Under the above situations, in order to modify the amino acid composition in alcoholic beverage components to produce alcoholic beverages having characteristic qualities, it is desired to provide a yeast in which the ability to assimilating amino acids is controlled.

The present inventors made exhaustive studies to solve the above problems and as a result, succeeded in identifying and isolating a gene encoding a tryptophan transporter which has more advantageous effects than the existing proteins from lager brewing yeast. Moreover, a yeast in which the obtained gene was transformed and expressed was produced to confirm that tryptophan assimilation can be controlled, thereby completing the present invention.

Thus, the present invention relates to a novel tryptophan transporter gene existing specifically in a lager brewing yeast, to a protein encoded by said gene, to a transformed yeast in which the expression of said gene is controlled, and to a method for producing alcoholic beverages by using a yeast in which the expression of said gene is controlled. More specifically, the present invention provides the following polynucleotides, a vector comprising said polynucleotide, a transformed yeast into which said vector is introduced, a method for producing alcoholic beverages using said transformed yeast, and the like.
(1) A polynucleotide selected from the group consisting of:
   (a) a polynucleotide comprising a polynucleotide consisting of the nucleotide sequence of SEQ ID NO:1;
   (b) a polynucleotide comprising a polynucleotide encoding a protein consisting of the amino acid sequence of SEQ ID NO:2;
   (c) a polynucleotide comprising a polynucleotide encoding a protein consisting of the amino acid sequence of SEQ ID NO:2 with one or more amino acids thereof being deleted, substituted, inserted and/or added, and having a tryptophan transporter activity;
   (d) a polynucleotide comprising a polynucleotide encoding a protein having an amino acid sequence having 60% or higher identity with the amino acid sequence of SEQ ID NO:2, and having a tryptophan transporter activity;
   (e) a polynucleotide comprising a polynucleotide which hybridizes to a polynucleotide consisting of a nucleotide sequence complementary to the nucleotide sequence of SEQ ID NO:1 under stringent conditions, and which encodes a protein having a tryptophan transporter activity; and
   (f) a polynucleotide comprising a polynucleotide which hybridizes to a polynucleotide consisting of a nucleotide sequence complementary to the nucleotide sequence of the polynucleotide encoding the protein of the amino acid sequence of SEQ ID NO:2 under stringent conditions, and which encodes a protein having a tryptophan transporter activity.
(2) The polynucleotide according to (1) above selected from the group consisting of:
   (g) a polynucleotide comprising a polynucleotide encoding a protein which consists of the amino acid sequence of SEQ ID NO: 2, or an amino acid sequence of SEQ ID NO: 2 wherein 1 to 10 amino acids thereof are deleted, substituted, inserted, and/or added, and which has a tryptophan transporter activity;
   (h) a polynucleotide comprising a polynucleotide encoding a protein, which has an amino acid sequence having 90% or higher identity with the amino acid sequence of SEQ ID NO: 2, and which has a tryptophan transporter activity; and
   (i) a polynucleotide comprising a polynucleotide consisting of the nucleotide sequence of SEQ ID NO: 1 or a polynucleotide which hybridizes to a polynucleotide consisting of a nucleotide sequence complementary to the nucleotide sequence of SEQ ID NO: 1 under high stringent conditions, and which has a tryptophan transporter activity.
(3) The polynucleotide of (1) above comprising a polynucleotide consisting of SEQ ID NO: 1.
(4) The polynucleotide of (1) above comprising a polynucleotide encoding a protein consisting of SEQ ID NO: 2.
(5) The polynucleotide of any one of (1) to (4) above, wherein the polynucleotide is DNA.
(6) A polynucleotide selected from the group consisting of:
   (j) a polynucleotide encoding RNA having a nucleotide sequence complementary to a transcript of the polynucleotide (DNA) according to (5) above;
   (k) a polynucleotide encoding RNA that represses the expression of the polynucleotide (DNA) according to (5) above through RNAi effect;
   (l) a polynucleotide encoding RNA having an activity of specifically cleaving a transcript of the polynucleotide (DNA) according to (5) above; and
   (m) a polynucleotide encoding RNA that represses the expression of the polynucleotide (DNA) according to (5) above through co-suppressiod effect.
(7) A protein encoded by the polynucleotide of any one of (1) to (5) above.
(8) A vector comprising the polynucleotide of any one of (1) to (5) above.
   (8a) The vector of (7) above, which comprises the expression cassette comprising the following components:
      (x) a promoter that can be transcribed in a yeast cell;
      (y) any of the polynucleotides described in (1) to (5) above linked to the promoter in a sense direction or in an antisense direction; and
      (z) a signal that can function in a yeast with respect to transcription termination and polyadenylation of a RNA molecule.
(9) A vector comprising the polynucleotide of (6) above.
(10) A yeast comprising the vector of (8) or (9) above.
(11) The yeast according to (10) above, wherein the tryptophan assimilation ability is increased owing to the introduction of the vector of (8) above.
(12) A yeast, wherein the expression of the polynucleotide (DNA) according to (5) above is repressed by introducing the vector of (9) above or by disrupting the gene related to the polynucleotide (DNA) according to (5) above.
(13) The yeast according to (11) above, wherein the tryptophan assimilation ability is improved by increasing the expression level of the protein of (7) above.
(14) A method for producing an alcoholic beverage using the yeast of any one of (10) to (13) above.
(15) The method for producing an alcoholic beverage of (14) above, wherein the brewed alcoholic beverage is a malt beverage.
(16) The method for producing an alcoholic beverage of (14) above, wherein the brewed alcoholic beverage is wine.
(17) An alcoholic beverage produced by the method of any one of (14) to (16) above.
(18) A method for assessing a test yeast for its tryptophan assimilation ability using a primer or a probe which is designed based on a nucleotide sequence of a tryptophan transporter gene having the nucleotide sequence of SEQ ID NO: 1.
   (18a) A method for selecting a yeast having a high or low tryptophan assimilation ability by using the method described in (18) above.
   (18b) A method for producing an alcoholic beverage (for example, beer) using the yeast selected by means of the method described in (18a) above.
(19) A method for assessing a test yeast for its tryptophan assimilation ability, comprising: culturing the test yeast; and measuring an expression level of a tryptophan transporter gene having the nucleotide sequence of SEQ ID NO: 1.
(20) A method for selecting a yeast, comprising: culturing test yeasts; quantifying the protein of (7) above or measuring the expression level of the tryptophan transporter gene having the nucleotide sequence of SEQ ID NO: 1; and selecting a test yeast having the production amount of the protein or the gene expression level according to the tryptophan assimilation ability of interest.
   (20a) A method for selecting a yeast, comprising: culturing test yeasts; measuring a tryptophan assimilation ability; and selecting a test yeast having a target tryptophan assimilation ability.
(21) The method for selecting a yeast according to (20) above, comprising: culturing a reference yeast and test yeasts; measuring the expression level of the tryptophan transporter gene having the nucleotide sequence of SEQ ID NO: 1 in each yeast; and selecting a test yeast having the gene expression higher or lower than that in the reference yeast.
(22) The method for selecting a yeast according to (20) above, comprising: culturing a reference yeast and test yeasts: quantifying the protein according to (7) above in each yeast; and selecting a test yeast having a larger or smaller amount of the protein than that in the reference yeast. That is, the method for selecting a yeast described in (20) above comprising: culturing plural yeasts; quantifying the protein of (7) above in each yeast; and selecting a test yeast having a large or small amount of the protein from them.
(23) A method for producing an alcoholic beverage comprising: conducting fermentation for producing an alcoholic beverage using the yeast according to any one of (10) to (13) above or a yeast selected by the method according to any one of (20) to (22) above; and adjusting the tryptophan content.

According to the method for producing an alcoholic beverage using the transformed yeast of the present invention, tryptophan assimilation is promoted. Therefore, the amino acid composition in alcoholic beverage components can be modified, and as a result, alcoholic beverages having characteristic qualities can be produced.

### BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract (sugar) consumption with time upon beer fermentation test. The horizontal axis represents fermentation time while the vertical axis represents apparent extract (sugar) concentration (w/w%).
Figure 3 shows the expression profile of non-ScTAT2 gene in yeasts upon beer fermentation test. The horizontal axis represents fermentation time while the vertical axis represents the intensity of detected signal.
Figure 4 shows the cell growth with time upon fermentation test. The horizontal axis represents fermentation time while the vertical axis represents optical density at 660 nm (OD660).
Figure 5 shows the extract (sugar) consumption with time upon beer fermentation test. The horizontal axis represents fermentation time while the vertical axis represents apparent extract (sugar) concentration (w/w%).

### BEST MODES FOR CARRYING OUT THE INVENTION

The present inventors conceived that it is possible to control tryptophan assimilation by adjusting the tryptophan transporter activity of yeasts. The present inventors have studied based on this conception and as a result, isolated and identified a non-ScTAT2 gene encoding a tryptophan transporter unique to lager brewing yeast based on the lager brewing yeast genome information mapped according to the method disclosed in Japanese Patent Application Laid-Open No. 2004-283169. The nucleotide sequence of the gene is represented by SEQ ID NO: 1. Further, an amino acid sequence of a protein encoded by the gene is represented by SEQ ID NO: 2.

### 1. Polynucleotide of the invention

First of all, the present invention provides (a) a polynucleotide comprising a polynucleotide consisting of the nucleotide sequence of SEQ ID NO:1; and (b) a polynucleotide comprising a polynucleotide encoding a protein consisting of the amino acid sequence of SEQ ID NO:2. The polynucleotide can be DNA or RNA.

The target polynucleotide of the present invention is not limited to the polynucleotide encoding a tryptophan transporter gene derived from lager brewing yeast described above and may include other polynucleotides encoding proteins having equivalent functions to said protein. Proteins with equivalent functions include, for example, (c) a protein consisting of an amino acid sequence of SEQ ID NO: 2 with one or more amino acids thereof being deleted, substituted, inserted and/or added and having a tryptophan transporter activity. Such proteins include a protein consisting of an amino acid sequence of SEQ ID NO: 2 with, for example, 1 to 100, 1 to 90, 1 to 80, 1 to 70, 1 to 60, 1 to 50, 1 to 40, 1 to 39, 1 to 38, 1 to 37, 1 to 36, 1 to 35, 1 to 34, 1 to 33, 1 to 32, 1 to 31, 1 to 30, 1 to 29, 1 to 28, 1 to 27, 1 to 26, 1 to 25, 1 to 24, 1 to 23, 1 to 22, 1 to 21, 1 to 20, 1 to 19, 1 to 18, 1 to 17, 1 to 16, 1 to 15, 1 to 14, 1 to 13, I to 12, 1 to 11, 1 to 10, 1 to 9, 1 to 8, 1 to 7, 1 to 6 (1 to several amino acids), 1 to 5, 1 to 4, 1 to 3, 1 to 2, or 1 amino acid residues thereof being deleted, substituted, inserted and/or added and having a tryptophan transporter activity, In general, the number of deletions, substitutions, insertions, and/or additions is preferably smaller. In addition, such proteins include (d) a protein having an amino acid sequence with about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2, and having a tryptophan transporter activity. In general, the percentage identity is preferably higher.

Tryptophan transporter activity may be measured, for example, by a method described in Mol Cell Biol. 14: 6597-6606, 1994.

Furthermore, the present invention also encompasses (e) a polynucleotide comprising a polynucleotide which hybridizes to a polynucleotide consisting of a nucleotide sequence complementary to the nucleotide sequence of SEQ ID NO: 1 under stringent conditions and which encodes a protein having a tryptophan transporter activity; and (f) a polynucleotide comprising a polynucleotide which hybridizes to a polynucleotide consisting of a nucleotide sequence complementary to a nucleotide sequence of a polynucleotide encoding a protein consisting of the amino acid sequence of SEQ ID NO: 2 under stringent conditions, and which encodes a protein having a tryptophan transporter activity.

Herein, "a polynucleotide that hybridizes under stringent conditions" refers to a polynucleotide, such as a DNA, obtained by a colony hybridization technique, a plaque hybridization technique, a southern hybridization technique or the like using all or a part of a polynucleotide consisting of a nucleotide sequence complementary to the nucleotide sequence of SEQ ID NO: 1 or a polynucleotide encoding the amino acid sequence of SEQ ID NO: 2 as a probe. The hybridization method may be a method described, for example, in MOLECULAR CLONING 3rd Ed., CURRENT PROTOCOLS IN MOLECULAR BIOLOGY, John Wiley & Sons 1987-1997, and so on.

The term "stringent conditions" as used herein may be any of low stringency conditions, moderate stringency conditions and high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting hybridized polynucleotide, such as DNA.

Other polynucleotides that can be hybridized include polynucleotides having about 60% or higher, about 70% or higher, 71% or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to a polynucleotide encoding the amino acid sequence of SEQ ID NO: 2 as calculated by homology search software, such as FASTA and BLAST using default parameters.

Identity between amino acid sequences or nucleotide sequences may be determined using algorithm BLAST by Karlin and Altschul *(*Proc. Natl. Acad. Sei. USA, 87: 2264-2268, 1990; Proc. Natl. Acad. Sci. USA, 90: 5873, 1993). Programs called BLASTN and BLASTX based on BLAST algorithm have been developed (Altschul SF et al., J. Mol. Biol. 215: 403, 1990). When a nucleotide sequence is sequenced using BLASTN, the parameters are, for example, score = 100 and word length = 12. When an amino acid sequence is sequenced using BLASTX, the parameters are, for example, score = 50 and word length = 3. When BLAST and Gapped BLAST programs are used, default parameters for each of the programs are employed.

The polynucleotide of the present invention includes (j) a polynucleotide encoding RNA having a nucleotide sequence complementary to a transcript of the polynucleotide (DNA) according to (5) above; (k) a polynucleotide encoding RNA that represses the expression of the polynucleotide (DNA) according to (5) above through RNAi effect; (1) a polynucleotide encoding RNA having an activity of specifically cleaving a transcript of the polynucleotide (DNA) according to (5) above; and (m) a polynucleotide encoding RNA that represses the expression of the polynucleotide (DNA) according to (5) above through co-suppression effect. These polynucleotides may be incorporated into a vector, which can be introduced into a cell for transformation to repress the expression of the polynucleotides (DNA) of (a) to (i) above. Thus, these polynucleotides may suitably be used when repression of the expression of the above polynucleotide (DNA) is preferable.

The phrase "polynucleotide encoding RNA having a nucleotide sequence complementary to the transcript of DNA" as used herein refers to so-called antisense DNA. Antisense technique is known as a method for repressing expression of a particular endogenous gene, and is described in various publications (see e.g., Hirajima and Inoue: New Biochemistry Experiment Course 2 Nucleic Acids IV Gene Replication and Expression (Japanese Biochemical Society Ed., Tokyo Kagaku Dozin Co., Ltd.) pp.319-347, 1993). The sequence of antisense DNA is preferably complementary to all or part of the endogenous gene, but may not be completely complementary as long as it can effectively repress the expression of the gene. The transcribed RNA has preferably 90% or higher, and more preferably 95% or higher complementarity to the transcript of the target gene. The length of the antisense DNA is at least 15 bases or more, preferably 100 bases or more, and more preferably 500 bases or more.

The phrase "polynucleotide encoding RNA that represses DNA expression through RNAi effect" as used herein refers to a polynucleotide for repressing expression of an endogenous gene through RNA interference (RNAi). The term "RNAi" refers to a phenomenon where when double-stranded RNA having a sequence identical or similar to the target gene sequence is introduced into a cell, the expressions of both the introduced foreign gene and the target endogenous gene are repressed. RNA as used herein includes, for example, double-stranded RNA that causes RNA interference of 21 to 25 base length, for example, dsRNA (double strand RNA), siRNA (small interfering RNA) or shRNA (short hairpin RNA). Such RNA may be locally delivered to a desired site with a delivery system such as liposome, or a vector that generates the double-stranded RNA described above may be used for local expression thereof. Methods for producing or using such double-stranded RNA (dsRNA, siRNA or shRNA) are known from many publications (see, e.g., Japanese National Phase PCT Laid-open Patent Publication No. 2002-516062; US 2002/086356A; Nature Genetics, 24(2), 180-183, 2000 Feb.; Genesis, 26(4), 240-244, 2000 April; Nature, 407:6802, 319-20, 2002 Sep. 21; Genes & Dev., Vo1.16, (8), 948-958, 2002 Apr.15; Proc. Natl. Acad. Sci. USA., 99(8), 5515-5520, 2002 Apr, 16; Science, 296(5567), 550-553, 2002 Apr. 19; Proc Natl. Acad. Sci. USA, 99:9, 6047-6052, 2002 Apr. 30; Nature Biotechnology, Vol.20 (5), 497-500, 2002 May; Nature Biotechnology, Vol. 20(5), 500-505, 2002 May; Nucleic Acids Res., 30:10, e46,2002 May 15).

The phrase "polynucleotide encoding RNA having an activity of specifically cleaving transcript of DNA" as used herein generally refers to a ribozyme. Ribozyme is an RNA molecule with a catalytic activity that cleaves a transcript of a target DNA and inhibits the function of that gene. Design of ribozymes can be found in various known publications (see, e.g., FEBS Lett. 228: 228, 1988; FEBS Lett. 239: 285, 1988; Nucl. Acids. Res. 17: 7059, 1989; Nature 323: 349, 1986; Nucl. Acids. Res. 19: 6751, 1991; Protein Eng 3: 733, 1990; Nucl. Acids Res. 19: 3875, 1991; Nucl. Acids Res. 19: 5125, 1991; Biochem Biophys Res Commun 186: 1271, 1992). In addition, the phrase "polynucleotide encoding RNA that represses DNA expression through co-suppression effect" refers to a nucleotide that inhibits functions of target DNA by "co-suppression".

The term "co-suppression" as used herein, refers to a phenomenon where when a gene having a sequence identical or similar to a target endogenous gene is transformed into a cell, the expressions of both the introduced foreign gene and the target endogenous gene are repressed. Design of polynucleotides having a co-suppression effect can also be found in various publications (see, e.g., Smyth DR: Curt. Biol. 7: R793, 1997, Martienssen R: Curr. Biol. 6: 810, 1996).

### 2. Protein of the present invention

The present invention also provides proteins encoded by any of the polynucleotides (a) to (i) above. A preferred protein of the present invention comprises an amino acid sequence of SEQ ID NO:2 with one or several amino acids thereof being deleted, substituted, inserted and/or added, and has a tryptophan transporter activity.

Such protein includes those having an amino acid sequence of SEQ ID NO: 2 with amino acid residues thereof of the number mentioned above being deleted, substituted, inserted and/or added and having a tryptophan transporter activity. In addition, such protein includes those having homology as described above with the amino acid sequence of SEQ ID NO: 2 and having a tryptophan transporter activity.

Such proteins may be obtained by employing site-directed mutation described, for example, in MOLECULAR CLONING 3rd Ed., CURRENT PROTOCOLS IN MOLECULAR BIOLOGY, Nuc. Acids. Res., 10: 6487 (1982), Proc. Natl. Acad. Sci. USA 79: 6409 (1982), Gene 34: 315 (1985), Nuc. Acids. Res., 13: 4431 (1985), Proc. Natl. Acad. Sci. USA 82: 488 (1985).

Deletion, substitution, insertion and/or addition of one or more amino acid residues in an amino acid sequence of the protein of the invention means that one or more amino acid residues are deleted, substituted, inserted and/or added at any one or more positions in the same amino acid sequence. Two or more types of deletion, substitution, insertion and addition may occur concurrently.

Hereinafter, examples of mutually substitutable amino acid residues are enumerated. Amino acid residues in the same group are mutually substitutable. The groups are provided below.
Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

### 3. Vector of the invention and yeast transformed with the vector

The present invention then provides a vector comprising the polynucleotide described above. The vector of the present invention is directed to a vector including any of the polynucleotides (DNA) described in (a) to (i) above. Generally, the vector of the present invention comprises an expression cassette including as components (x) a promoter that can transcribe in a yeast cell; (y) a polynucleotide (DNA) described in any of (a) to (i) above that is linked to the promoter in a sense or antisense direction; and (z) a signal that functions in the yeast with respect to transcription termination and polyadenylation of RNA molecule. According to the present invention, in order to highly express the protein of the invention described above upon brewing alcoholic beverages (e.g., beer) described below, these polynucleotides are introduced in the sense direction to the promoter to promote expression of the polynucleotide (DNA) described in any of (a) to (i) above. Further, in order to repress the expression of the above protein of the invention upon brewing alcoholic beverages (e.g., beer) described below, these polynucleotides are introduced in the antisense direction to the promoter to repress the expression of the polynucleotide (DNA) described in any of (a) to (i) above. In order to repress the expression of the above protein of the invention, the polynucleotide may be introduced into vectors such that the polynucleotide of any of the (j) to (m) above is to be expressed. According to the present invention, the target gene (DNA) may be disrupted to repress the expression of the polynucleotide (DNA) described above or the expression of the protein described above. A gene may be disrupted by adding or deleting one or more bases to or from a region involved in expression of the gene product in the target gene, for example, a coding region or a promoter region, or by deleting these regions entirely. Such disruption of gene may be found in known publications (see, e.g., Proc. Natl. Acad. Sci. USA, 76, 4951(1979), Methods in Enzymology, 101, 202 (1983), and Japanese Patent Application Laid-Open No. 6-253826).

A vector introduced in the yeast may be any of a multicopy type (YEp type), a single copy type (YCp type), or a chromosome integration type (YIp type). For example, YEp24 (J. R. Broach et al., EXPERIMENTAL MANIPULATION OF GENE EXPRESSION, Academic Press, New York, 83, 1983) is known as a YEp type vector, YCp50 (M. D. Rose et al., Gene 60: 237, 1987) is known as a YCp type vector, and YIp5 (K. Struhl et al., Proc. Natl. Acad. Sci. USA, 76: 1035, 1979) is known as a YIp type vector, all of which are readily available.

Promoters/terminators for adjusting gene expression in yeast may be in any combination as long as they function in the brewery yeast and they are not influenced by constituents in fermentation broth. For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F. Tuite et al., EMBO J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUP1) (Marin et al., Proc. Natl. Acad. Sci. USA, 81, 337 1984) or a cerulenin-resistant gene (fas2m, PDR4) (Junji Inokoshi et al., Biochemistry, 64, 660, 1992; and Hussain et al., Gene, 101: 149, 1991, respectively) may be used.

A vector constructed as described above is introduced into a host yeast. Examples of the host yeast include any yeast that can be used for brewing, for example, brewery yeasts for beer, wine and sake. Specifically, yeasts such as genus *Saccharomyces* may be used. According to the present invention, a lager brewing yeast, for example, *Saccharomyces pastorianus* W34/70, etc., *Saccharomyces carlsbergensis* NCYC453 or NCYC456, etc., or *Saccharomyces cerevisiae* NBRC1951, NBRC1952, NBRC1953 or NBRC1954, etc., may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan may also be used but not limited thereto. In the present invention, lager brewing yeasts such as *Saccharomyces pastorianus* may be used preferably.

A yeast transformation method may be a generally used known method. For example, methods that can be used include but not limited to an electroporation method *(*Meth. Enzym., 194: 182 (1990)), a spheroplast method *(*Proc. Natl. Acad. Sci. USA, 75: 1929(1978)), a lithium acetate method *(*J. Bacteriology, 153: 163 (1983)), and methods described in Proc. Natl. Acad. Sci. USA, 75: 1929 (1978), METHODS IN YEAST GENETICS, 2000 Edition: A Cold Spring Harbor Laboratory Course Manual, and the like.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali metal ion, preferably lithium ion at a concentration of about 1 to 2 M. After the cell is left to stand at about 30°C for about 60 minutes, it is left to stand with DNA to be introduced (about 1 to 20 µg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant.

Other general cloning techniques may be found, for example, in MOLECULAR CLONING 3rd Ed., and METHODS IN YEAST GENETICS, A LABORATORY MANUAL (Cold Spring Harbor Laboratory Press, Cold Spring Harbor, NY).

### 4. Method of producing alcoholic beverages according to the present invention and alcoholic beverages produced by the method

The vector of the present invention described above is introduced into a yeast suitable for brewing a target alcoholic product. This yeast can be used to produce an alcoholic product having a characteristic amino acid composition. In addition, yeasts to be selected by the yeast assessment method of the present invention described below can also be used, The target alcoholic beverages include, for example, but not limited to beer, beer-taste beverages such as sparkling liquor *(happoushu),* wine, whisky, sake and the like. Further, according to the present invention, desired alcoholic beverages with reduced tryptophan level can be produced using brewery yeast in which the expression of the target gene was suppressed, if needed. That is to say, desired kind of alcoholic beverages with controlled (elevated or reduced) level of tryptophan can be produced by controlling (elevating or reducing) production amount of tryptophan using yeasts into which the vector of the present invention described above was introduced, yeasts in which expression of the polynucleotide (DNA) of the present invention described above was suppressed or yeasts selected by the yeast assessment method of the invention described below for fermentation to produce alcoholic beverages.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages whose fermentation period is shortened. Thus, according to the present invention, alcoholic beverages can be produced using the existing facility without increasing the cost.

### 5. Yeast assessment method of the invention

The present invention relates to a method for assessing a test yeast for its tryptophan assimilation ability by using a primer or a probe designed based on a nucleotide sequence of a tryptophan transporter gene having the nucleotide sequence of SEQ ID NO:1. General techniques for such assessment method are known and are described in, for example, WO01/040514, Japanese Laid-Open Patent Application No. 8-205900 or the like. This assessment method is described below.

First, genome of a test yeast is prepared. For this preparation, any known method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the tryptophan transporter gene, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of a polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the tryptophan assimilation ability of the yeast as determined by whether the molecular weight of the amplified product is a size that contains the DNA molecule of the specific part. In addition, by analyzing the nucleotide sequence of the amplified product, the above-described ability may be predicted and/or assessed more precisely.

Moreover, in the present invention, a test yeast is cultured to measure an expression level of the tryptophan transporter gene having the nucleotide sequence of SEQ ID NO: 1 to assess the test yeast for its tryptophan assimilation ability. In measuring an expression level of the tryptophan transporter gene, the test yeast is cultured and then mRNA or a protein resulting from the transcription of the tryptophan transporter gene is quantified. The quantification of mRNA or protein may be carried out by employing a known technique. For example, mRNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003). In addition, it is possible to predict the expression level of the above-described gene in a test yeast by measuring the tryptophan concentration in a fermentation liquor which is obtained when culturing the test yeast.

Furthermore, test yeasts are cultured and expression levels of the tryptophan transporter gene having the nucleotide sequence of SEQ ID NO: 1 are measured to select a test yeast with the gene expression level corresponding to the target tryptophan assimilation ability, thereby selecting a yeast favorable for brewing desired alcoholic beverages. In addition, a reference yeast and test yeasts may be cultured so as to measure and compare the expression level of the gene in each of the yeasts, thereby selecting a favorable test yeast. More specifically, for example, a reference yeast and test yeasts are cultured and an expression level of the tryptophan transporter gene having the nucleotide sequence of SEQ ID NO: 1 is measured in each yeast. By selecting a test yeast with the gene expressed higher or lower than that in the reference yeast, a yeast suitable for brewing desired alcoholic beverages can be selected.

Alternatively, test yeasts are cultured and a yeast with a higher or lower tryptophan assimilation ability is selected, thereby selecting a yeast suitable for brewing desired alcoholic beverages.

In these cases, the test yeasts or the reference yeast may be, for example, a yeast introduced with the vector of the invention described above, an artificially mutated yeast or a naturally mutated yeast. Assessment of the tryptophan assimilation ability can be carried out, for example, by analyzing the amino acid composition of beer after completion of fermentation using an amino acid analyzer (e.g., L-8800 high-speed amino acid analyzer, manufactured by Hitachi, Ltd.) and a standard amino acid analytical column (P/N855-3506, manufactured by Hitachi, Ltd.) to assess the tryptophan concentration in the amino acid composition. The mutation treatment may employ any methods including, for example, physical methods such as ultraviolet irradiation and radiation irradiation, and chemical methods associated with treatments with drugs such as EMS (ethylmethane sulphonate) and N-methyl-N-nitrosoguanidine *(see, e.g.,* Yasuji Oshima Ed., BIOCHEMISTRY EXPERIMENTS vol. 39, Yeast Molecular Genetic Experiments, pp. 67-75, JSSP).

In addition, examples of yeasts used as the reference yeast or the test yeasts include any yeast that can be used for brewing, for example, brewery yeasts for beer, wine, sake and the like. More specifically, yeasts such as genus *Saccharomyces* may be used. According to the present invention, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954, etc., may be used. Further, whisky yeasts such as *Saccharomyces cerevisiae* NCYC90, wine yeasts such as wine yeasts #1, 3 and 4 from the Brewing Society of Japan; and sake yeasts such as sake yeast #7 and 9 from the Brewing Society of Japan may also be used but not limited thereto. In the present invention, lager brewing yeasts such as *Saccharomyces pastorianus* may preferably be used. The reference yeast and the test yeasts may be selected from the above yeasts in any combination.

### EXAMPLES

Hereinafter, the present invention will be described in more detail with reference to working examples. The present invention, however, is not limited to the examples described below.

### Example 1: Cloning of Novel Tryptophan Transporter (non-ScTAT2) Gene

A specific novel tryptophan transporter gene (non-ScTAT2) (SEQ ID NO: 1) from a lager brewing yeast was found, as a result of a search utilizing the comparison database described in Japanese Patent Application Laid-Open No. 2004-283169. Based on the acquired nucleotide sequence information, primers non-ScTAT2_F (SEQ ID NO: 3) and non-ScTAT2_R (SEQ ID NO: 4) were designed to amplify the full-length genes, respectively. PCR was carried out using chromosomal DNA of a genome sequencing strain, *Saccharomyces pastorianus* Weihenstephan 34/70 strain, as a template to obtain DNA fragments including the full-length gene of non-ScTAT2.

The thus-obtained non-ScTAT2 gene fragment was inserted into pCR2.1-TOPO vector (Invitrogen) by TA cloning. The nucleotide sequences of non-ScTAT2 gene were analyzed according to Sanger's method (F. Sanger, *Science,* 214: 1215, 1981) to confirm the nucleotide sequence.

### Example 2: Analysis of Expression of non-ScTAT2 Gene during Beer Fermentation

A beer fermentation test was conducted using a lager brewing yeast, *Saccharomyces pastorianus* W34/70 strain and then mRNA extracted from yeast cells during fermentation was analyzed by a DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

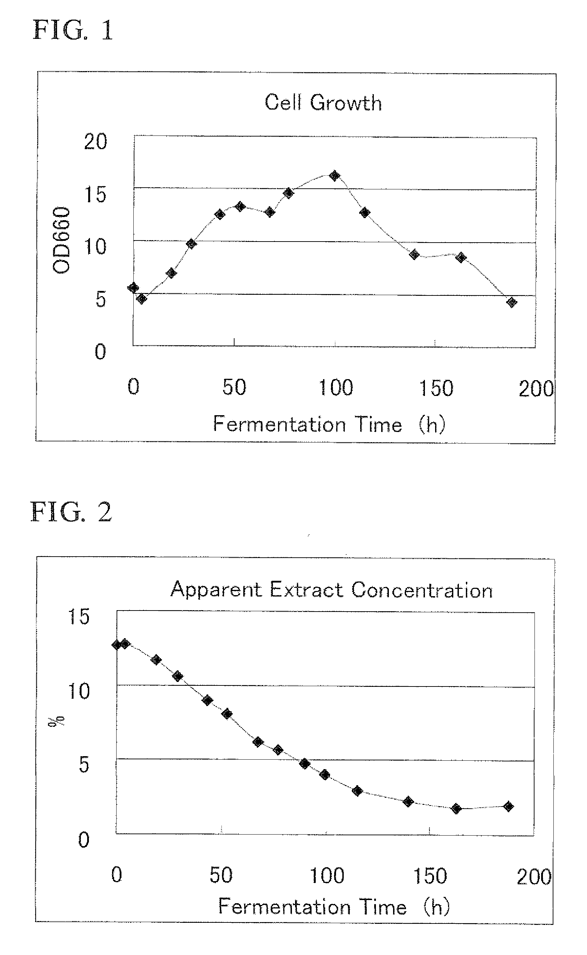

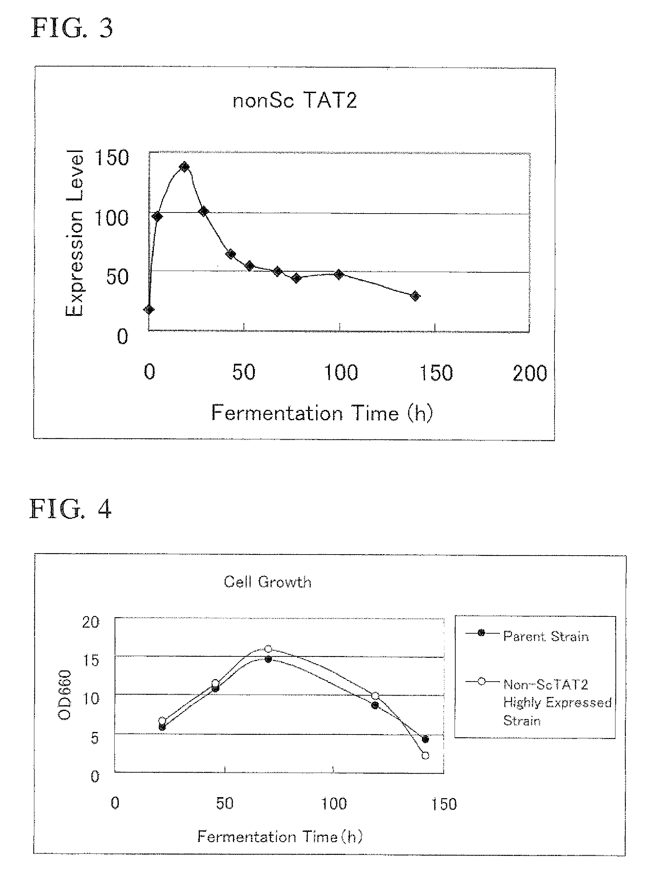

Sampling of fermentation liquor was performed with time, and variation with time of yeast growth amount (Fig. 1) and apparent extract concentration (Fig. 2) was observed. Simultaneously, sampling of yeast cells was performed, and the prepared mRNA was subjected to be biotin-labeted and was hybridized to a beer yeast DNA microarray described in Japanese Patent Application Laid-Open No. 2004-283169. The signal was detected using GCOS; GeneChip Operating Software 1.0 (manufactured by Affymetrix Co.). Expression pattern of non-ScTAT2 gene is shown in Figure 3. As a result, it was confirmed that non-ScTAT2 gene was expressed in the general beer fermentation.

### Example 3: Construction of non-ScTAT2 Gene Highly Expressed Strain

The non-ScTAT2/pCR2.1-TOPO described in Example 1 was digested using the restriction enzymes SacI and NotI so as to prepare a DNA fragment containing the entire length of the protein-encoding region. This fragment was ligated to pYCGPYNot treated with the restriction enzymes SacI and NotI, thereby constructing the non-ScTAT2 high expression vector non-ScTAT2/pYCGPYNot. pYCGPYNot is the YCp-type yeast expression vector. The inserted gene is highly expressed by the pyruvate kinase gene PYK1 promoter. The geneticin-resistant gene G418^{r} is included as the selection marker in the yeast, and the ampicillin-resistant gene Amp^{r} is included as the selection marker in *Escherichia coli.*

Using the high expression vector prepared by the above method, the strain *Saccharomyces pasteurianus* Weihenstephaner 34/70 was transformed by the method described in Japanese Patent Application Laid-open No. H7-303475. The transformant was selected in a YPD plate culture (1% yeast extract, 2% polypeptone, 2% glucose, 2% agar) containing 300 mg/L of geneticin.

### Example 4: Measurement of Assimilation of Amino Acids in Beer Fermentation

A fermentation test was carried out under the following conditions using the parent strain (34/70 strain) and the non-ScTAT2 highly expressed strain obtained in Example 3.

| | |
|---|---|
| Wort extract concentration | 12 % |
| Wort content | 1 L |
| Wort dissolved oxygen concentration | approx. 7 ppm |
| Fermentation temperature | 12°C (fixed) |
| Yeast pitching rate | 5 g wet yeast cells/L of wort |

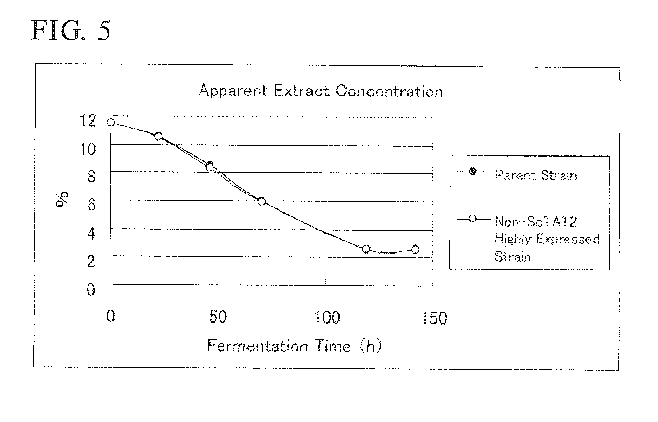

The fermentation broth was sampled over time, and variation with time of the yeast growth rate (OD660) (FIG. 4) and the amount of extract consumption (FIG. 5) was determined. Further, when the amino acid composition of beer after completion of fermentation was measured using the L-8800 high-speed amino acid analyzer (manufactured by Hitachi, Ltd.) and the standard amino acid analytical column P/N855-3506 (manufactured by Hitachi, Ltd.), the amino acid composition of the beer produced using the non-ScTAT2-highly expressed strain showed a different characteristic, wherein tryptophan was more decreased compared to the amino acid composition of the beer produced using the parent strain, as shown in Table 1.

**Table 1**

| Amino Acid Concentration (mM) | Parent Strain | non-ScTAT2-highly expressed strain |
|---|---|---|
| Tryptophan | 0.1843 | 0.1135 |

### Example 5: Disruption of non-ScTAT2 Gene

According to the publication (Goldstein et al., yeast. 15 1541 (1999)), PCR using a plasmid including a drug-resistant marker (pFA6a (*G418^{r}*), pAG25 (*nat1*) or pAG32 (*hph*)) as a template is conducted to prepare a fragment for gene disruption.

With the prepared fragment for gene disruption, W34/70 strain or spore cloning strain (W34/70-2) is transformed. The transformation is performed in accordance with the method described in Japanese Patent Application Laid-Open No. H07-303475. The concentrations of the drugs for selection are 300 mg/L for geneticin and 50 mg/L of nourseothricin, respectively.

### Example 6: Measurement of Assimilation of Amino Acids in Beer Fermentation

A fermentation test is carried out under the following conditions using the parent strain and the non-ScTAT2-disrpted strain obtained in Example 5.

| | |
|---|---|
| Wort extract concentration | 12% |
| Wort content | 1 L |
| Wort dissolved oxygen concentration | approx. 7 ppm |
| Fermentation temperature | 12°C (fixed) |
| Yeast pitching rate | 5 g wet yeast cells/L of wort |

The fermentation broth is sampled over time, and variation with time of the yeast growth rate (OD660) and the amount of extract consumption is determined. Further, the amino acid composition of beer after completion of fermentation is measured using the L-8800 high-speed amino acid analyzer (manufactured by Hitachi, Ltd.) and the standard amino acid analytical column P/N855-3506 (manufactured by Hitachi, Ltd.) to determine the amount of assimilation of amino acids.

### INDUSTRIAL APPLICABILITY

The inventive method of producing alcoholic beverages may allow for production of alcoholic beverages having a characteristic amino acid composition by adjusting the tryptophan content, because the tryptophan assimilation ability of yeast can be controlled by the method.

## Claims

1. A polynucleotide selected from the group consisting of:
(a) a polynucleotide comprising a polynucleotide consisting of the nucleotide sequence of SEQ ID NO:1;
(b) a polynucleotide comprising a polynucleotide encoding a protein consisting of the amino acid sequence of SEQ ID NO:2;
(c) a polynucleotide comprising a polynucleotide encoding a protein consisting of the amino acid sequence of SEQ ID NO:2 with one or more amino acids thereof being deleted, substituted, inserted and/or added, and having a tryptophan transporter activity;
(d) a polynucleotide comprising a polynucleotide encoding a protein having an amino acid sequence having 60% or higher identity with the amino acid sequence of SEQ ID NO:2, and having a tryptophan transporter activity;
(e) a polynucleotide comprising a polynucleotide which hybridizes to a polynucleotide consisting of a nucleotide sequence complementary to the nucleotide sequence of SEQ ID NO:1 under stringent conditions, and which encodes a protein having a tryptophan transporter activity; and
(f) a polynucleotide comprising a polynucleotide which hybridizes to a polynucleotide consisting of a nucleotide sequence complementary to the nucleotide sequence of the polynucleotide encoding the protein of the amino acid sequence of SEQ ID NO:2 under stringent conditions, and which encodes a protein having a tryptophan transporter activity.

2. The polynucleotide according to claim 1 selected from the group consisting of:
(g) a polynucleotide comprising a polynucleotide encoding a protein which consists of the amino acid sequence of SEQ ID NO: 2, or an amino acid sequence of SEQ ID NO: 2 wherein 1 to 10 amino acids thereof are deleted, substituted, inserted, and/or added, and which has a tryptophan transporter activity;
(h) a polynucleotide comprising a polynucleotide encoding a protein, which has an amino acid sequence having 90% or higher identity with the amino acid sequence of SEQ ID NO: 2, and which has a tryptophan transporter activity; and
(i) a polynucleotide comprising a polynucleotide consisting of the nucleotide sequence of SEQ ID NO: 1 or a polynucleotide which hybridizes to a polynucleotide consisting of a nucleotide sequence complementary to the nucleotide sequence of SEQ ID NO: 1 under high stringent conditions, and which has a tryptophan transporter activity.

3. The polynucleotide of claim 1 comprising a polynucleotide consisting of SEQ ID NO: 1.

4. The polynucleotide of claim 1 comprising a polynucleotide encoding a protein consisting of SEQ ID NO: 2.

5. The polynucleotide of any one of claims 1 to 4, wherein the polynucleotide is DNA.

6. A polynucleotide selected from the group consisting of:
(j) a polynucleotide encoding RNA having a nucleotide sequence complementary to a transcript of the polynucleotide (DNA) according to claim 5;
(k) a polynucleotide encoding RNA that represses the expression of the polynucleotide (DNA) according to claim 5 through RNAi effect;
(l) a polynucleotide encoding RNA having an activity of specifically cleaving a transcript of the polynucleotide (DNA) according to claim 5; and
(m) a polynucleotide encoding RNA that represses the expression of the polynucleotide (DNA) according to claim 5 through co-suppression effect.

7. A protein encoded by the polynucleotide of any one of claims 1 to 5.

8. A vector comprising the polynucleotide of any one of claims 1 to 5.

9. A vector comprising the polynucleotide of claim 6.

10. A yeast comprising the vector of claim 8 or 9.

11. The yeast according to claim 10, wherein the tryptophan assimilation ability is increased owing to the introduction of the vector of claim 8.

12. A yeast, wherein the expression of the polynucleotide (DNA) according to claim 5 is repressed by introducing the vector of claim 9 or by disrupting the gene related to the polynucleotide (DNA) according to claim 5.

13. The yeast according to claim 11, wherein the tryptophan assimilation ability is improved by increasing the expression level of the protein of claim 7.

14. A method for producing an alcoholic beverage using the yeast of any one of claims 10 to 13.

15. The method for producing an alcoholic beverage of claim 14, wherein the brewed alcoholic beverage is a malt beverage.

16. The method for producing an alcoholic beverage of claim 14, wherein the brewed alcoholic beverage is wine.

17. An alcoholic beverage produced by the method of any one of claims 14 to 16.

18. A method for assessing a test yeast for its tryptophan assimilation ability using a primer or a probe which is designed based on a nucleotide sequence of a tryptophan transporter gene having the nucleotide sequence of SEQ ID NO: 1.

19. A method for assessing a test yeast for its tryptophan assimilation ability, comprising: culturing the test yeast; and measuring an expression level of a tryptophan transporter gene having the nucleotide sequence of SEQ ID NO: 1.

20. A method for selecting a yeast, comprising: culturing test yeasts; quantifying the protein of claim 7 or measuring the expression level of the tryptophan transporter gene having the nucleotide sequence of SEQ ID NO: 1; and selecting a test yeast having the production amount of the protein or the gene expression level according to the tryptophan assimilation ability of interest.

21. The method for selecting a yeast according to claim 20, comprising: culturing a reference yeast and test yeasts; measuring the expression level of the tryptophan transporter gene having the nucleotide sequence of SEQ ID NO: 1 in each yeast; and selecting a test yeast having the gene expression higher or lower than that in the reference yeast.

22. The method for selecting a yeast according to claim 20, comprising: culturing a reference yeast and test yeasts; quantifying the protein according to claim 7 in each yeast; and selecting a test yeast having a larger or smaller amount of the protein than that in the reference yeast.

23. A method for producing an alcoholic beverage comprising: conducting fermentation for producing an alcoholic beverage using the yeast according to any one of claims 10 to 13 or a yeast selected by the method according to any one of claims 20 to 22; and adjusting the tryptophan content.


SEQUENCE LISTING

<110> Suntory Limited

<120> Tryptophan transporter gene and use thereof

<130> G07-0145

<140> PCT/JP2006/317703
<141> 2006-08-31

<150> JP2005-253271
<151> 2005-09-01

<160> 4

<170> PatentIn version 3.1

<210> 1
<211> 1779
<212> DNA
<213> yeast

<400> 1
atggctggtg actttattcc ttccaccacg tatccgagtg aggagcttaa ggaacggaaa 60
tccacatccg aattcgtagg ctacaaatcc aagcagttaa catcctcttc gtcgcacaat 120
tcggacacat ctcatcacga tgacaactat caatatagca acaggaacat cttccagcgg 180
tgcgtcgact ctttcaagcc gcccctggac ggttcgtttg acacaggtaa cctgaaaagg 240
acactgaagc ccaggcattt gatcatgatt gccatcgggg gcagtatagg gaccggtctg 300
tttgtcggta gtggtaaagc catcgcggag ggcggtcctc tgggtgtcgt cctaggttgg 360
accattgccg gctcgcaaat catcggaacc atccatgggt tgggtgaaat cacagtacgg 420
ttccccgttg tgggcgcgtt tgccaattat ggtacaaggt ttttggaccc cagtataagt 480
tttgttgttt ctaccatata cgtgctacag tggttcttcg ttttgccctt ggaaatcatt 540
gctgcagcca tgaccgtgca gtactggaac tccgccatcg accccgtagt ttgggtggcc 600
atcttttacg tcatcattgt atccataaac ctgtttggtg caagaggttt cggcgaggct 660
gaattcgcat tttccaccat caaggcaata acggtttgcg ggttcatcat attatgcatc 720
gtccttattt ttggaggagg acccgaccat gaatacattg gcgccaaata ctggtacgat 780
cctggttgct tggccaacgg gtttcccggt gttttgtccg tgcttgttgt tgcctcgtat 840
tccctgggtg gtattgaaat gacatgtttg gcatcgggtg aaacggatcc taagggtcta 900

ccaagcgcta tcaagcaagt gttttggaga atcttgtttt tctttttggt gtcgttgacg 960
ctggtcggat tcctggtccc atatacaaac caaaatctgc tcggtggttc atccgtagac 1020
aactcgccct tcgtgatcgc catcaaactg caccatatca aagttttgcc atccattgtc 1080
aacgtggtca ttctaatcag tgttttgagt gtggggaact cctgcatctt cgcttcgagt 1140
agaacactgt gttccatggc ccatcaaggc cttattccgt ggtggtttgg ttacatcgac 1200
cgtgccggca gaccgctagt tggcattatg gcaaactcgc tgttcgggtt actagccttt 1260
ctggtcaaat caggttccat ggcagacatc ttcaattggc tcatggccat tgctggtcta 1320
gccacgtgta tagtctggtt gagtatcaat ttgtcgcata tcagatttag aatggcaatg 1380
aaggcccagg ggaaaaaact ggatgagctg gagtttgtca gtgctgtcgg gacatgggga 1440
tctgcgtact ccgcattaat caactgcctg atcttgatag cccagtttta ctgttcttta 1500
tggcccattg gtggctggaa agatagcaaa gatagagcaa agatattctt ccaaaactac 1560
ctctgcgctt taatcatgtt attcatattc gtcacccaca agatttacta taagtgccaa 1620
acaggccaat ggtgggggat taaagctcta aaggacatcg atctagaaac tgatcgcaaa 1680
aacatcgata tcgagattgt caagcaggaa atcgccgaaa agaagatgtt cttggattca 1740
tgtccgtggt atgtcagaca cttccattac tggtgctag 1779

<210> 2
<211> 592
<212> PRT
<213> yeast

<400> 2

Met Ala Gly Asp Phe Ile Pro Ser Thr Thr Tyr Pro Ser Glu Glu Leu
1               5                   10                  15

Lys Glu Arg Lys Ser Thr Ser Glu Phe Val Gly Tyr Lys Ser Lys Gln
            20                  25                  30

Leu Thr Ser Ser Ser Ser His Asn Ser Asp Thr Ser His His Asp Asp
        35                  40                  45

Asn Tyr Gln Tyr Ser Asn Arg Asn Ile Phe Gln Arg Cys Val Asp Ser
    50                  55                  60

Phe Lys Pro Pro Leu Asp Gly Ser Phe Asp Thr Gly Asn Leu Lys Arg
65                  70                  75                  80

Thr Leu Lys Pro Arg His Leu Ile Met Ile Ala Ile Gly Gly Ser Ile

                85                  90                  95

Gly Thr Gly Leu Phe Val Gly Ser Gly Lys Ala Ile Ala Glu Gly Gly
            100                 105                 110

Pro Leu Gly Val Val Leu Gly Trp Thr Ile Ala Gly Ser Gln Ile Ile
        115                 120                 125

Gly Thr Ile His Gly Leu Gly Glu Ile Thr Val Arg Phe Pro Val Val
    130                 135                 140

Gly Ala Phe Ala Asn Tyr Gly Thr Arg Phe Leu Asp Pro Ser Ile Ser
145                 150                 155                 160

Phe Val Val Ser Thr Ile Tyr Val Leu Gln Trp Phe Phe Val Leu Pro
                165                 170                 175

Leu Glu Ile Ile Ala Ala Ala Met Thr Val Gln Tyr Trp Asn Ser Ala
            180                 185                 190

Ile Asp Pro Val Val Trp Val Ala Ile Phe Tyr Val Ile Ile Val Ser
        195                 200                 205

Ile Asn Leu Phe Gly Ala Arg Gly Phe Gly Glu Ala Glu Phe Ala Phe
    210                 215                 220

Ser Thr Ile Lys Ala Ile Thr Val Cys Gly Phe Ile Ile Leu Cys Ile
225                 230                 235                 240

Val Leu Ile Phe Gly Gly Gly Pro Asp His Glu Tyr Ile Gly Ala Lys
                245                 250                 255

Tyr Trp Tyr Asp Pro Gly Cys Leu Ala Asn Gly Phe Pro Gly Val Leu
            260                 265                 270

Ser Val Leu Val Val Ala Ser Tyr Ser Leu Gly Gly Ile Glu Met Thr
        275                 280                 285

Cys Leu Ala Ser Gly Glu Thr Asp Pro Lys Gly Leu Pro Ser Ala Ile

290                 295                 300

Lys Gln Val Phe Trp Arg Ile Leu Phe Phe Phe Leu Val Ser Leu Thr
305                 310                 315                 320

Leu Val Gly Phe Leu Val Pro Tyr Thr Asn Gln Asn Leu Leu Gly Gly
                325                 330                 335

Ser Ser Val Asp Asn Ser Pro Phe Val Ile Ala Ile Lys Leu His His
            340                 345                 350

Ile Lys Val Leu Pro Ser Ile Val Asn Val Val Ile Leu Ile Ser Val
        355                 360                 365

Leu Ser Val Gly Asn Ser Cys Ile Phe Ala Ser Ser Arg Thr Leu Cys
    370                 375                 380

Ser Met Ala His Gln Gly Leu Ile Pro Trp Trp Phe Gly Tyr Ile Asp
385                 390                 395                 400

Arg Ala Gly Arg Pro Leu Val Gly Ile Met Ala Asn Ser Leu Phe Gly
                405                 410                 415

Leu Leu Ala Phe Leu Val Lys Ser Gly Ser Met Ala Asp Ile Phe Asn
            420                 425                 430

Trp Leu Met Ala Ile Ala Gly Leu Ala Thr Cys Ile Val Trp Leu Ser
        435                 440                 445

Ile Asn Leu Ser His Ile Arg Phe Arg Met Ala Met Lys Ala Gln Gly
    450                 455                 460

Lys Lys Leu Asp Glu Leu Glu Phe Val Ser Ala Val Gly Thr Trp Gly
465                 470                 475                 480

Ser Ala Tyr Ser Ala Leu Ile Asn Cys Leu Ile Leu Ile Ala Gln Phe
                485                 490                 495

Tyr Cys Ser Leu Trp Pro Ile Gly Gly Trp Lys Asp Ser Lys Asp Arg

500                 505                 510

Ala Lys Ile Phe Phe Gln Asn Tyr Leu Cys Ala Leu Ile Met Leu Phe
        515                 520                 525

Ile Phe Val Thr His Lys Ile Tyr Tyr Lys Cys Gln Thr Gly Gln Trp
    530                 535                 540

Trp Gly Ile Lys Ala Leu Lys Asp Ile Asp Leu Glu Thr Asp Arg Lys
545                 550                 555                 560

Asn Ile Asp Ile Glu Ile Val Lys Gln Glu Ile Ala Glu Lys Lys Met
                565                 570                 575

Phe Leu Asp Ser Cys Pro Trp Tyr Val Arg His Phe His Tyr Trp Cys
            580                 585                 590

<210>  3
<211>  40
<212>  DNA
<213>  primer

<400>  3
gagctcatag cggccatggc tggtgacttt attccttcca                          40

<210>  4
<211>  42
<212>  DNA
<213>  primer

<400>  4
ggatcctatg cggccgctcg ttatgcatta gattgtgccg ga                       42